Europäisches Patentamt

European Patent Office

Office européen des brevets

(19)

(11) Veröffentlichungsnummer: **0 377 070**

**A1**

## (12) EUROPÄISCHE PATENTANMELDUNG

(21) Anmeldenummer: 89102458.0

(22) Anmeldetag: 13.02.89

(51) Int. Cl.5: **A61B 6/03**

(30) Priorität: 03.01.89 EP 89100048

(43) Veröffentlichungstag der Anmeldung:
**11.07.90 Patentblatt 90/28**

(84) Benannte Vertragsstaaten:
**DE FR GB**

(71) Anmelder: **Siemens Aktiengesellschaft**
**Wittelsbacherplatz 2**
**D-8000 München 2(DE)**

(72) Erfinder: **Kalender, Willi, Dr. Dipl.-Phys.**
**Auf der Höh 5**
**D-8521 Kleinseebach(DE)**

(54) **Computertomograph.**

(57) Bei einem Computertomographen mit einem eine Meßöffnung (3) umschließenden Anodenring (4), auf dem der Fokus einer Röntgenstrahlenquelle umläuft, soll bei hoher Röntgenröhrenleistung eine optimale Bildqualität erzielt werden.

Der Strahlendetektor (13) ist nicht wie beim Stand der Technik als feststehender Detektorring, sondern wie bei den Computertomographen der dritten Generation als um einen Fokuspunkt gekrümmtes, teilkreisförmiges Detektorarray ausgebildet, das synchron mit dem Fokus um eine gemeinsame Achse (8) rotiert. Der Strahlendetektor kann zusammen mit einer rotierenden Kathode im Vakuumgefäß angeordnet sein.

EP 0 377 070 A1

FIG 2

## Computertomograph

Die Erfindung betrifft einen Computertomographen mit einem eine Meßöffnung umschließenden Anodenring in einem Vakuumgefäß, auf dem der Fokus einer Röntgenstrahlenquelle zur Durchstrahlung eines Untersuchungsobjektes mit einem fächerförmigen Röntgenstrahlenbündel unter verschiedenen Richtungen umläuft, mit einem aus einer Reihe von Detektorelementen bestehenden Strahlendetektor zum Empfang der aus dem Untersuchungsobjekt austretenden Röntgenstrahlung und mit einem Rechner zur Bildung der Schwächungswerte vorbestimmter Bildpunkte in der untersuchten Schicht.

Aufgrund der Ausbildung der Röntgenstrahlenquelle als geschlossener Anodenring, der die Meßöffnung umschließt, ist eine gute Wärmeabfuhr und damit ein hoher Röntgenröhrenstrom möglich. Dies erlaubt die schnell aufeinanderfolgende Abtastung vieler paralleler Schichten.

Bei einem bekannten Computertomographen dieser Art (US 4 158 142) ist der Strahlendetektor als feststehender Detektorring ausgebildet, der gegenüber dem Anodenring seitlich versetzt angeordnet ist. Die Fächerebene des Röntgenstrahlenbündels liegt nicht senkrecht zu seiner Drehachse, so daß das Röntgenstrahlenbündel nach dem Austritt aus dem Untersuchungsobjekt auf dem Strahlendetektor auftreffen kann. Es führt bei der Abtastung des Untersuchungsobjektes eine Taumelbewegung aus. Die versetzte Anordnung von Anodenring und Strahlendetektor ergibt eine ungünstige Schichtgeometrie und verschlechtert die Bildqualität.

Der Erfindung liegt die Aufgabe zugrunde, einen Computertomographen der eingangs genannten Art so auszubilden, daß gegenüber dem Stand der Technik eine Verbesserung der Schichtgeometrie und damit bei hoher Röntgenröhrenleistung die Aufnahme einer Vielzahl paralleler Schichten des Untersuchungsobjektes innerhalb kürzester Zeit bei bester Bildqualität ermöglicht ist.

Diese Aufgabe ist erfindungsgemäß dadurch gelöst, daß der Strahlendetektor ein um einen Fokuspunkt gekrümmtes, teilkreisförmiges Array ist, das synchron mit dem Fokus um eine gemeinsame Achse rotiert. Bei dem erfindungsgemäßen Computertomographen ist der Strahlendetektor wie bei einem Computertomographen der dritten Generation ausgebildet, jedoch einer Röntgenröhre mit einem die Meßöffnung vollständig umschließenden, also ruhenden Anodenring zugeordnet. Dies ermöglicht eine hohe Röntgenröhrenleistung und höchste Bildqualität aufgrund eines mit dem Fokus synchron mitführbaren, kollimierten Strahlendetektors.

Eine besonders zweckmäßige Weiterbildung der Erfindung besteht darin, daß der Strahlendetektor im Vakuumgefäß angeordnet und mit einer rotierenden Kathode verbunden ist. Bei dieser Ausführungsform wird die rotierende Kathode über Schleifringe im Vakuumgefäß mit Strom versorgt. Die Signale der Detektorelemente des Strahlendetektors können ebenfalls über Schleifringe im Vakuumgefäß abgenommen werden. Die Ausrichtung des Strahlendetektors auf den Fokus ist in jeder Stellung der Kathode und des Strahlendetektors automatisch gegeben. Eine besondere Synchronisation zwischen dem rotierenden Strahlendetektor und der rotierenden Kathode ist deshalb nicht erforderlich.

Die Erfindung ist nachfolgend anhand der Zeichnung näher erläutert. Es zeigen:

Fig. 1 und 2 zwei verschiedene Ansichten eines ersten Ausführungsbeispieles nach der Erfindung, und

Fig. 3 und 4 zwei verschiedene Ansichten eines zweiten Ausführungsbeispieles nach der Erfindung.

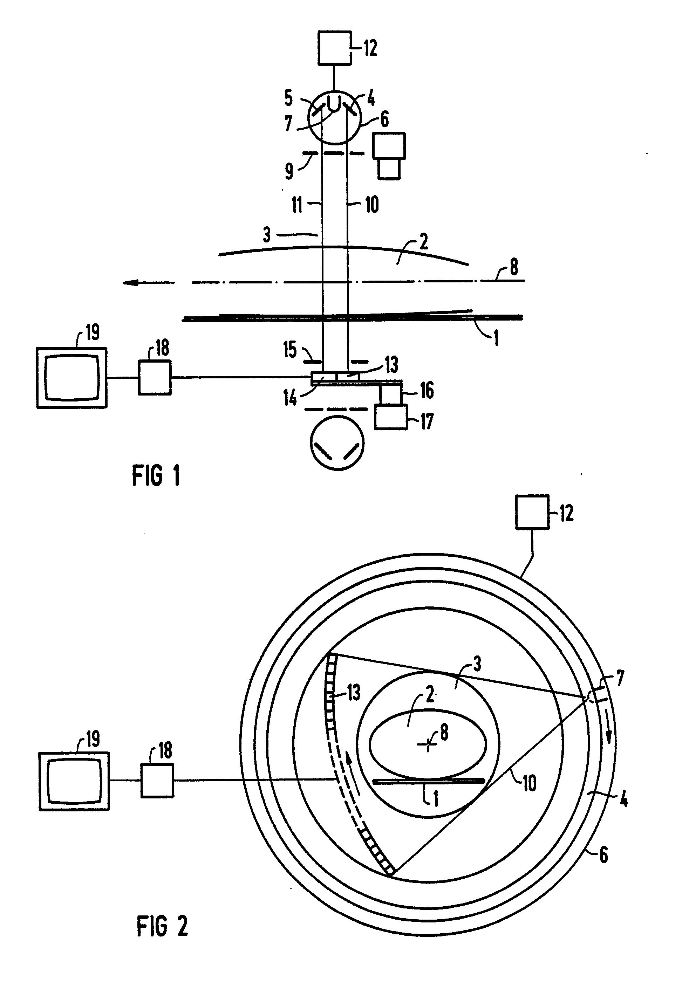

In den Fig. 1 und 2 ist eine Patientenliege 1 dargestellt, auf der ein Patient 2 ruht, von dem eine Vielzahl paralleler Schichtbilder angefertigt werden soll. Die Patientenliege 1 befindet sich in der Meßöffnung 3 eines Computertomographen, welcher eine Röntgenstrahlenquelle mit zwei Anodenringen 4, 5 aufweist, von denen in der Fig. 2 nur der Anodenring 4 sichtbar ist. Die Anodenringe 4, 5 befinden sich in einem Vakuumgefäß 6. In dem Vakuumgefäß 6 rotiert eine geheizte Kathode 7, deren Elektronenstrahlung auf dem jeweiligen Fokus auf den Anodenringen 4, 5 auftrifft. Der Fokus auf den Anodenringen 4, 5 rotiert demgemäß um eine Achse 8. Eine Primärstrahlenblende 9 erlaubt die Einblendung von zwei fächerförmigen Röntgenstrahlenbündeln 10, 11, von denen in der Fig. 2 nur das Röntgenstrahlenbündel 10 sichtbar ist. Der Patient 2 wird demgemäß gleichzeitig von den beiden Röntgenstrahlenbündeln 10, 11 unter verschiedenen Richtungen durchstrahlt. Die Speisung der Röntgenröhre 4 bis 7 erfolgt von einem Röntgengenerator 12.

Die Erfassung der aus dem Patienten 2 austretenden Röntgenstrahlung erfolgt durch zwei parallele Detektorarrays 13, 14, von denen in der Fig. 2 nur das Detektorarray 13 sichtbar ist. Die Detektorarrays 13, 14 sind teilkreisförmig um den jeweiligen Fokuspunkt auf den Anodenringen 4, 5 gekrümmt und rotieren synchron mit dem jeweiligen Fokus auf den Anodenringen 4, 5, so daß sie dem Fokus immer um 180° gegenüberliegen. Die empfangene Strahlung wird durch eine Blende 15 eingeblendet, die mit den Detektorarrays 13, 14 rotiert.

Zur Drehung der Detektorarrays 13, 14 ist ein Ring 16 vorgesehen, der um die Achse 8 in einem ringförmigen Lager 17 drehbar gelagert ist. Die Komponenten 16, 17 sind in Figur 2 nicht dargestellt.

Die während der Drehung des Fokus auf den Anodenringen 4, 5 gewonnenen Meßwerte der Detektorelemente der Detektorarrays 13, 14 werden einem Rechner 18 zugeführt, der daraus die Schwächungswerte vorbestimmter Bildpunkte in der untersuchten Schicht berechnet und auf einem Sichtgerät 19 bildlich wiedergibt.

Aufgrund der Tatsache, daß eine hohe Röntgenröhrenleistung möglich ist, kann der Patient 2 kontinuierlich durch die Meßöffnung 3 hindurchgeschoben werden, so daß in schneller Folge eine Vielzahl von parallelen Schichten des Patienten 2 abgetastet wird. Auf diese Weise ist eine dreidimensionale Bilderzeugung in kurzer Zeit bei hoher Bildqualität möglich. Die Aufnahmezeit ist durch die gleichzeitige Abtastung zweier paralleler Schichten gemäß Fig. 1 verkürzt. Es ist jedoch auch möglich, nur eine Schicht abzutasten, wobei sich immer noch eine kurze Aufnahmezeit für ein bestimmtes Patientenvolumen ergibt.

Aufgrund der Tatsache, daß in kürzester Zeit die Schwächungswerte eines bestimmten Volumens des patienten 2 vorliegen, ist es nicht nur möglich, parallele Schichtbilder, sondern Schichtbilder in beliebiger Schräglage zur Achse 8 zu erzeugen.

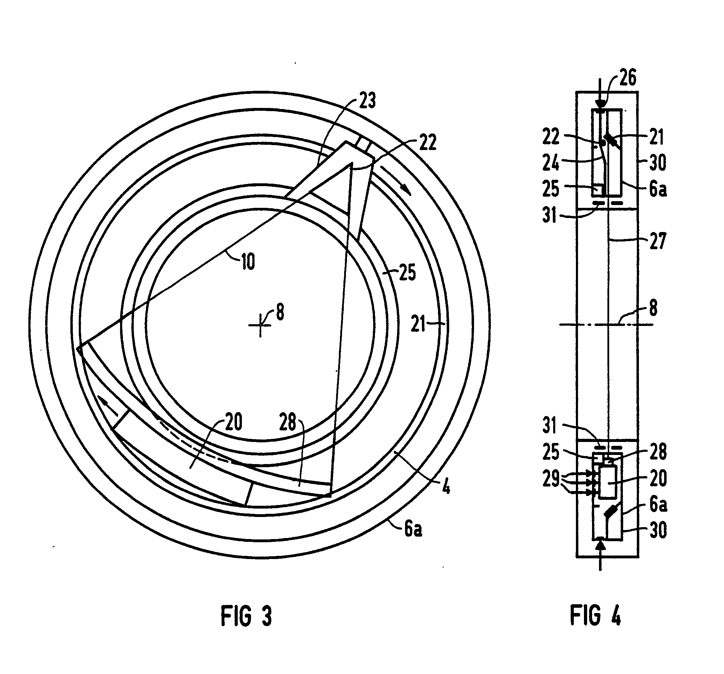

Bei dem Ausführungsbeispiel gemäß den Fig. 3 und 4 sind Teile, die mit Teilen der Fig. 1 und 2 gleich sind, mit den gleichen Bezugszeichen bezeichnet. Im Anschluß an den Strahlendetektor 28 ist noch ein Datenerfassungssystem 20 dargestellt. Der Computertomograph gemäß den Fig. 3 und 4 enthält nur einen einzigen Anodenring 21, dem eine rotierende Kathode 22 zugeordnet ist. Die Kathode 22 ist zusammen mit einer Primärstrahlenblende 23 (in Fig. 4 nicht gezeigt) über einen Halter 24 an einem Ring 25 im Vakuumgefäß 6a befestigt. Der Ring 25 ist zusammen mit der Kathode 22 im Vakuumgefäß 6a drehbar. Die Energie wird der Kathode 22 über eine Schleifringanordnung 26 zugeführt. Der Zentralstrahl der Röntgenstrahlung ist mit 27 bezeichnet. Auf dem Ring 25 sind auch der Strahlendetektor 28 und das Datenerfassungssystem 20 befestigt. Die Daten werden über eine Schleifringanordnung 29 abgeführt. Das Vakuumgefäß 6a ist in einem Gehäuse 30 angeordnet, in dem auch noch eine Blende 31 liegt.

Zur Abtastung des Patienten 2 unter verschiedenen Richtungen wird der Ring 25 gedreht, wodurch die Kathode 22, die Primärstrahlenblende 23, der Strahlendetektor 28 und das Datenerfassungssystem 20 in starrer Ausrichtung zueinander gedreht werden.

Die Figuren zeigen einen kompakten Aufbau bei äußerst geringer Tiefe des Vakuumgefäßes 6, 6a. Damit ergibt sich eine gute Zugänglichkeit zum Patienten 2 und ein großer Schwenkbereich für das Gehäuse um eine horizontale Achse zur Einstellung der Lage der untersuchten Schicht.

## Ansprüche

1. Computertomograph mit einem ein Meßöffnung (3) umschließenden Anodenring (4, 5, 21) ein einem Vakuumgefäß (6, 6a), auf dem der Fokus einer Röntgenstrahlenquelle zur Durchstrahlung eines Untersuchungsobjektes (2) mit einem fächerförmigen Röntgenstrahlenbündel (10, 11) unter verschiedenen Richtungen umläuft, mit einem aus einer Reihe von Detektorelementen bestehenden Strahlendetektor (13, 14, 28) zum Empfang der aus dem Untersuchungsobjekt (2) austretenden Röntgenstrahlung und mit einem Rechner (18) zur Bildung der Schwächungswerte vorbestimmter Bildpunkte in der untersuchten Schicht, **dadurch gekennzeichnet,** daß der Strahlendetektor (13, 14, 28) ein um einen Fokuspunkt gekrümmtes, teilkreisförmiges Array ist, das synchron mit dem Fokus um eine gemeinsame Achse (8) rotiert.

2. Computertomograph nach Anspruch 1, **dadurch gekennzeichnet,** daß mindestens ein Anodenring (4, 5, 21) in einem gemeinsamen Vakuumgefäß (6, 6a) vorgesehen ist, wodurch mindestens ein Röntgenstrahlenbündel (10, 11) zur Durchstrahlung verschiedener Schichten erzeugt wird.

3. Computertomograph nach Anspruch 1 oder 2, **dadurch gekennzeichnet,** daß der Strahlendetektor (28) im Vakuumgefäß (6a) angeordnet und mit einer rotierenden Kathode (22) verbunden ist.

4. Computertomograph nach Anspruch 3, **dadurch gekennzeichnet,** daß der Strahlendetektor (28) und die Kathode (22) sowie eine Strahlenblende (23) auf einem drehbaren Ring (25) im Vakkumgefäß (6a) angeordnet sind.

GR 89 P 3003 E 01

FIG 1

FIG 2

FIG 3

FIG 4

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 647 220 (EMI LTD)<br>* Figuren 1-4; Seite 9, Zeile 1 - Seite 18, Zeile 19 * | 1-4 | A 61 B 6/03 |
| X | DE-A-2 708 612 (NIHON DENSHI K.K.)<br>* Figur 1; Seite 8, Zeile 16 - Seite 11, Zeile 23 * | 1,2 | |
| A | WO-A-8 000 754 (WESTERN ELECTRIC)<br>* Figur 1; Seite 3, Zeile 2 - Seite 5, Zeile 7 * | 1-3 | |
| A | GB-A-2 034 149 (TOKYO SHIBAURA DENKI)<br>* Figuren 1,2,6-9,12; Seite 2, Zeile 16 - Seite 3, Zeile 85; Seite 4, Zeilen 1-89 * | 2 | |

RECHERCHIERTE SACHGEBIETE (Int. Cl.4)

A 61 B

Der vorliegende Recherchenbericht wurde für alle Patentansprüche erstellt

| Recherchenort | Abschlußdatum der Recherche | Prüfer |
|---|---|---|
| DEN HAAG | 31-07-1989 | CHEN A.H. |